# EUROPÄISCHE PATENTSCHRIFT

(11) **EP 1 246 784 B1**
(45) Veröffentlichungstag und Bekanntmachung des Hinweises auf die Patenterteilung: **19.10.2005**
(21) Anmeldenummer: 01900150.2
(22) Anmeldetag: 10.01.2001
(51) Int. Cl.: C07B 49/00, C07C 41/30, C07C 25/13, C07D 495/04

(54) **REAKTION VON CARBONYLVERBINDUNGEN MIT METALLORGANISCHEN REAGENZIEN**
REACTION OF CARBONYL COMPOUNDS WITH ORGANOMETALLIC REAGENTS
REACTION DE COMPOSES CARBONYLE AVEC DES REACTIFS ORGANO-METALLIQUES

(30) Priorität: 14.01.2000 DE 10001317
(43) Veröffentlichungstag der Anmeldung: 09.10.2002
(73) Patentinhaber: Merck Patent GmbH, 64293 Darmstadt (DE)
(72) Erfinder: KOOP, Ulrich, 64380 Rossdorf (DE); KRUMMRADT, Holger, 64319 Pfungstadt (DE); SCHWARZ, Michael, 64331 Weiterstadt (DE); STOLDT, Jöran, 64331 Weiterstadt (DE); ECKSTEIN, Jürgen, 64380 Rossdorf (DE); ZEHNER, Stefan, 64850 Schaafheim (DE); MELICHAR, Wolfgang, 64285 Darmstadt (DE)
(86) Internationale Anmeldenummer: PCT/EP2001/000248
(87) Internationale Veröffentlichungsnummer: WO 2001/051434

(56) Entgegenhaltungen:
- WO-A-99/22857
- DE-A- 4 411 101

## Beschreibung

Die Erfindung betrifft ein neues Verfahren zur Durchführung von Reaktionen von Carbonylverbindungen mit metallorganischen Reagenzien, insbesondere mit Grignardreagenzien.

Die Selektivität bzw. Qualität einer chemischen Reaktion wird unter anderem stark durch die exakte Kontrolle von Temperatur und Verweilzeit beeinflußt.

Ein Beispiel für eine Reaktion von Carbonylverbindungen mit metallorganischen Reagenzien wird in der Patentanmeldung DE 44 11 101 A1 ('Verbessertes Verfahren zur Herstellung eines D-(+)-Biotin-Zwischenproduktes") beschrieben. Es handelt sich dabei um folgende Reaktion:

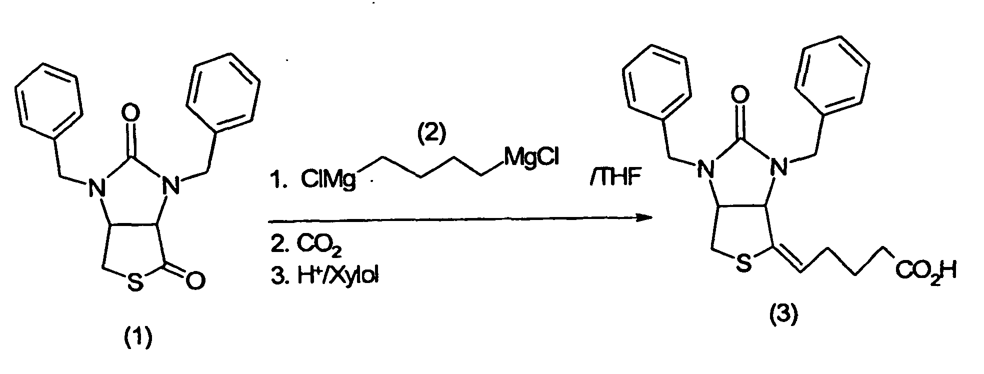

Zur Durchführung dieser Reaktion wird nach diesem Verfahren in einer Rührwerksapparatur 1,4-(Dichlormagnesium)-butan in Tetrahydrofuran vorgelegt. Bei -25 bis -15°C wird in Tetrahydrofuran gelöstes (+)-cis-1,3-Dibenzyl-hexahydro-1 H-thieno[3,4d]imidazol-2,4-dion (1) zudosiert. Anschließend wird das durch die Reaktion entstandene Zwischenprodukt mit Kohlendioxid weiter umgesetzt. Nach einem Austausch des Lösungsmittels (Tetrahydrofuran wird durch Xylol ersetzt) wird mit 30%iger Schwefelsäure bei T > 50°C dehydratisiert. Zur Abtrennung der entstandenen Nebenprodukte folgen dann pH-kontrollierte Extraktionen.

Die Ausbeute dieser Reaktion liegt trotz intensiver Optimierungsversuche zur Zeit im Produktionsmaßstab bei ca. 65 - 75%. Nachteilig ist bei dieser Art der Reaktionsdurchführung die Entstehung von verschiedenen Nebenprodukten in größeren Mengen, deren Bildung durch sehr lange Verweilzeiten stark begünstigt wird (τ ≈ 12 h).

In WO 99/22857 A1 wird versucht, chemische Reaktionen unter konstanten Bedingungen durchzuführen, indem mit einem Mikromischer, bestehend aus einem geraden, dünnen Kanal, in den die Edukte gasförmig und/oder als Lösungen in einem spitzen Winkel eingeleitet werden und zur Reaktion gebracht werden. In dem Kanal, der einen Durchmesser im Bereich von 10 bis 10 000 µm aufweisen kann, erfolgt die Reaktion, indem insbesondere sich die durchfließenden Zweiphasensysteme durch Diffusion an den Grenzflächen vermischen und reagieren. In der Beschreibung werden entsprechende Beispiele zu Gas-Flüssig- und Flüssig-Flüssig-Reaktionen angesprochen. Weiterhin wird in dieser Anmeldung zwar darauf hingewiesen, dass verschiedenste Reaktionstypen und u. a. auch Grignardreaktionen in diesem Reaktortyp durchgeführt werden können. Das Problem von Reaktionen mit Edukt-Lösungen, in denen Feststoffteilchen auftreten können, ist dagegen nicht erkannt worden. Es wird weder in einem Beispiel noch in der Beschreibung darauf eingegangen, in welcher Weise Verstopfungen des dünnen Reaktionskanals durch gebildete Partikel vermieden werden können, zumal beispielsweise die zur Durchführung von Grignardreaktionen eingesetzten Lösungen sind nicht frei von Partikeln sind sondern im günstigsten Fall feinste Suspensionen darstellen, noch wird durch ein Beispiel gezeigt, in welcher Weise dieses üblicherweise bei der Durchführung von entsprechenden Reaktionen im Mikromischer auftretende Problem gelöst werden kann.

Aufgabe der vorliegenden Erfindung ist es daher, ein Verfahren zur Verfügung zu stellen, durch das Reaktionen von Carbonylverbindungen mit metallorganischen Verbindungen in einfacher Weise mit verbesserter Selektivität mit einer höheren Raum-Zeit-Ausbeute durchgeführt werden können.

Die Lösung der Aufgabe erfolgt durch ein Verfahren zur Durchführung von Reaktionen von Carbonylverbindungen mit metallorganischen Reagenzien, welches dadurch gekennzeichnet ist, daß die Lösungen
a) eines metallorganischen Reagenzes ausgewählt aus der Gruppe der Grignardreagenzien und lithiumorganischen Verbindungen gelöst, in einem aprotischen nukleophilen Lösungsmittel und
b) einer Carbonylverbindung aus der Gruppe der aliphatischen, cycloaliphatischen und heterocyclischen Ketone, gelöst in einem aprotischen nukleophilen Lösungsmittel
vorgelegt werden und bei einer konstanten Temperatur zwischen - 40 bis +120 °C, vorzugsweise bis 60°C, in einer Verweilzeit von τ < 10 s unter Kontrolle des Drucks,
gegebenenfalls nachdem die Eduktlösungen aus a) und b) mit Pumpen durch eine Filtrationseinheit geführt worden sind, in einem kontinuierlich betriebenen Mini/Mikroreaktor intensiv vermischt werden.

Als temperierter Mini/Mikroreaktor wird in dem erfindungsgemäßen Verfahren ein plattenförmigen Mikromischer verwendet, worin gegebenenfalls die Edukte in einen 180°-T-Stück oder in einem Winkel von 30 bis 150° zusammengeführt werden und eine intensive Durchmischung gegebenenfalls in zopfförmigen dünnen Leitungen erfolgt.

Gegebenenfalls wird das resultierende Vorprodukt durch eine nachgeschaltete, sehr dünne, temperierte Verweilstrecke geführt.

Als metallorganisches Reagenz sind im Verfahren sowohl Grignardreagenzien als auch lithiumorganische Verbindungen einsetzbar. Dementsprechend können Verbindungen aus der Gruppe 1,4-Dichlormagnesiumbutan, 3,4,5-Trifluor-1-brommagnesiumbenzol und n-Butyllithium eingesetzt werden.

Als Carbonylverbindung kann eine Verbindung ausgewählt aus der Gruppe der aliphatischen, cycloaliphatischen und heterocyclischen Ketone eingesetzt werden.

Besonders vorteilhaft ist das erfindungsgemäße Verfahren zur Umsetzung von metallorganischen Verbindungen mit Carbonylverbindungen ausgewählt aus der Gruppe 4-(Pentylcyclohexyl-1)-cyclohexan-4-on (4) und (+)-cis-1,3-Dibenzyl-hexahydro-1H-thieno[3,4d]imidazol-2,4-dion.

Als Lösungsmittel für die metallorganische Verbindung aber auch für die Carbonylverbindung kann ein aprotisches nukleophiles Lösungsmittel verwendet mungskanälen in der Größe von 1 bis 1000 µm zu verstehen. Hingegen hat ein "Minireaktor" Strömungsquerschnitte im Bereich > 1000 µm.

Hierdurch läßt sich die Reaktion bei intensiver Vermischung mit definierter Verweilzeit durchführen. Gleichzeitig ist unter diesen Bedingungen gewährleistet, daß das Reaktionsgemisch während der Umsetzung im Reaktor unter nahezu idealen Bedingungen konstant bei einer festgelegten Temperatur gehalten wird, und zwar je nach eingesetzten Edukten zwischen -40 bis gegebenenfalls +120 °C.

Erfindungsgemäß werden bei der Herstellung des oben angeführten Zwischenprodukts für die Herstellung von Biotin die Lösungen der Edukte Grignard-Reagenz und Thiolacton in einem kontinuierlich betriebenen Mini/Mikroreaktor bei definierter Temperatur intensiv vermischt. Das daraus resultierende Vorprodukt wird, nachdem es gegebenenfalls nach dem Austritt aus dem Mikroreaktor durch eine sich anschließende sehr dünne temperierte Verweilzeitstrecke geführt worden ist, in einem sich anschließenden geeigneten Reaktor mit Kohlendioxid carboxyliert. Die Prozeßparameter Druck, Temperatur und Massenströme werden dabei an verschiedenen Stellen gemessen und geregelt

Das beschriebene Verfahren eignet sich allgemein für die Umsetzung von metallorganischen Reagenzien mit Carbonylverbindungen, da die Prozeßparameter Konzentrationsverhältnis, Druck, Temperatur und Fluß bzw. Verweilzeit exakt eingestellt werden können. Mit der Möglichkeit der exakten Reaktionsführung und der Verwendung kleiner Reaktorvolumina wird auf diese Weise ein hohes Sicherheitsniveau erreicht.

Zur Durchführung der oben aufgeführten Reaktion zur Herstellung des Zwischenprodukts von Biotin wird folgendermaßen vorgegangen: Das Thiolacton (1) wird in Tetrahydrofuran gelöst mit der Grignard-Verbindung (2) wahlweise im Mikro- oder Minireaktor mit nachfolgender Verweilzeitstrecke (Verweilzeit: τ < 10 s) kontinuierlich umgesetzt. Das anfallende Vorprodukt wird direkt in eine mit gasförmigem Kohlendioxid gefüllte Rührwerksapparatur geleitet. Die weitere Aufarbeitung des Rohprodukts entspricht dem Batch-Verfahren wie es auch in DE 44 11 101 A1 beschrieben ist. Die analytischen Daten (NMR, DC) stimmen mit denen der Batch-Reaktion überein.

In verschiedenen Versuchen, bei denen als Reaktionsparameter die Verweilzeit im Mikroreaktor variiert wurde, wurden überraschend hohe Ausbeuten des erwünschten Produkts erzielt. Es wurden beispielsweise folgende Ausbeuten erhalten (Fluß 2 l/h pro Edukt, C_{Grignard} : C_{Thiolacton} = 2 : 1 bis 1.5 : 1):
96.5% bei -10°C
90.1 % bei 40°C
92% bei 15°C

Diese Ergebnisse wurden bereits unter noch nicht optimierten Bedingungen erzielt, so daß noch weitere Steigerungen zu erwarten sind.

Zur Durchführung im Technikumsmaßstab wurden Mini-Statikmischer eingesetzt wie in DE 19746583 (Fig. 1) beschrieben. Hierbei wurden beispielsweise folgende Ausbeuten erreicht:
94.0% bei -25°C, 2 l/h pro Edukt
95.6% bei -10°C, 2 l/h pro Edukt
93.7% bei 5°C, 2 l/h pro Edukt

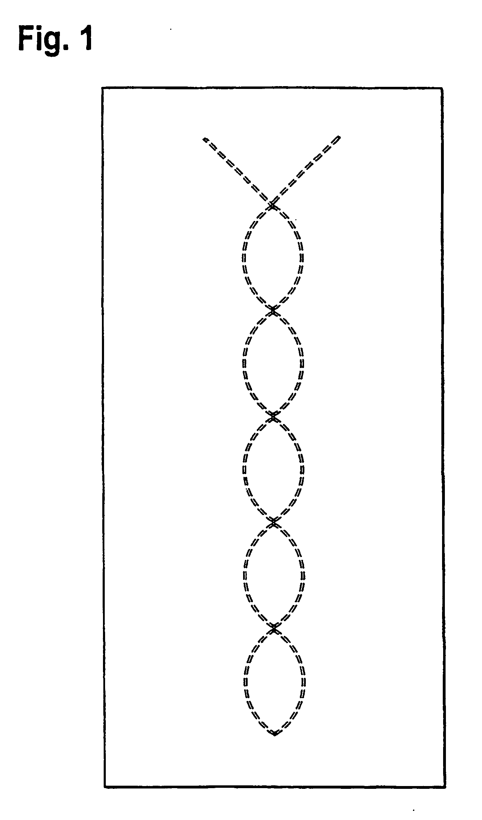

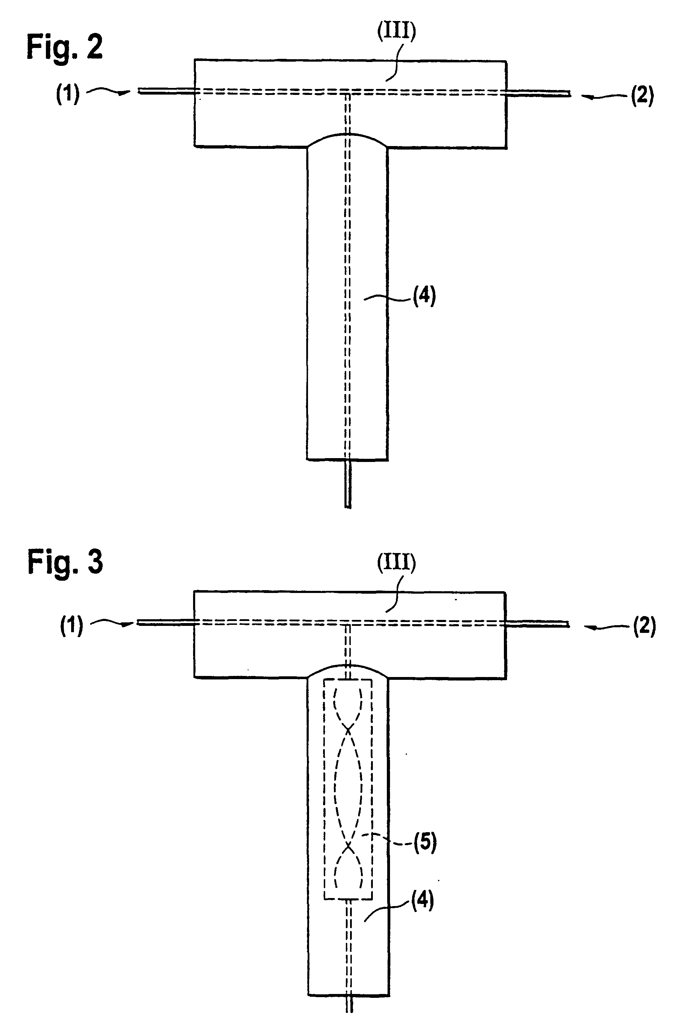

Aber auch mit einem sehr vereinfachten Mischer, in dem die Edukte lediglich mit Hilfe von dünnen Leitungen mit einem Innendurchmesser von 2mm in einem temperierten T-Stück zusammengeführt und gegebenenfalls durch eine sich anschließende Verweilstrecke in Form einer dünnen Leitung mit gleichem Durchmesser geführt werden (Fig. 2), werden wesentlich höhere Ausbeuten als bisher möglich erzielt:
92.6% bei ca. 5-10°C, 5 l/h pro Edukt, T-Stück mit 2 mm Innendurchmesser
   88.2 % bei 28-33°C, 30 kg/h pro Edukt, T-Stück mit 2 mm Innendurchmesser
84% bei 5-24°C, 5-30 kg/h pro Edukt, T-Stück mit 4 mm Innendurchmesser und nachgeschaltetem Statikmischer (hier wurden in einem Ansatz meherere Versuchsparameter getestet).

Auch wenn die Edukte in einem Winkel zwischen 30° und 150° zusammengeführt werden, läßt sich eine signifikante Ausbeuteerhöhung feststellen.

Wenn die Edukte in einem statischen Mischer nicht nur in einem T-Stück zusammengeführt werden sondern das entstehende Reaktionsgemisch auch noch durch zopfförmige Leitungen geführt wird (Fig.3), können noch weitere Ausbeutesteigerungen erzielt werden wie Versuche gezeigt haben.

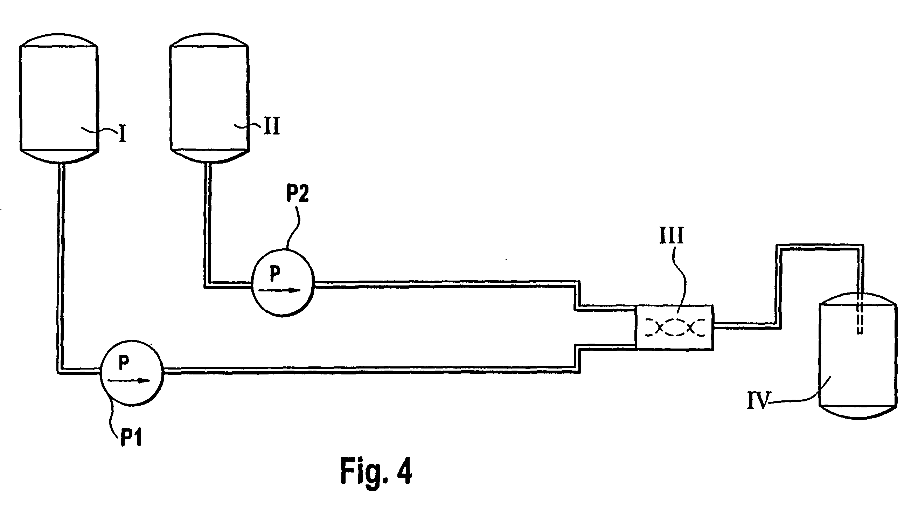

In Figur 4 ist ein grundlegender Aufbau einer Anlage zur Umsetzung von Carbonylverbindungen mit metallorganischen Reagenzien gezeigt. In den beiden Vorratstanks, bzw. Rührbehältern (I) und (II) werden die Edukte (1) und (2) vorgelegt. Druck- und Temperatursensoren sowie Durchflußmessungen sind an verschiedenen Stellen eingebaut, um den Prozeß optimal steuern zu können. Weiterhin ist es sinnvoll, wenn Vortemperierstrecken in die Eduktleitungen vor dem Reaktor (III) integriert sind. Weiterhin ist es möglich mehrere Reaktoren hintereinander zu schalten, wodurch auch mehrstufige Reaktionen durchgeführt werden können. Zur Förderung der Edukte aus den Vorratstanks (I) und (II) in den Reaktor (III) sind in der modellhaften Anlage der Fig. 4 Pumpen zwischengeschaltet. Nach dem Austritt aus dem statischen Mini/Mikroreaktor wird das Reaktionsgemisch in einen nachgeschalteten Vorratstank bzw. Rührbehältern (IV) geleitet, in dem eine nachgeschaltete Reaktion, wie z. B. die Umsetzung mit CO₂ erfolgen kann.

Im einzelnen kann eine geeignete Anlage aber auch so aufgebaut sein, daß dem Reaktor eine Pumpe nachgeschaltet ist, um einen unerwünschten Druckaufbau zu vermeiden.

Im besonderen wurden Versuche im Technikumsmaßstab mit folgendem Versuchsaufbau durchgeführt:
Zur Darstellung der Grignard-Verbindung wurde eine Standard-Rührwerksapparatur verwendet, die auch als Vorratsgefäß diente. Als Vorratsgefäß für die Thiolacton-Lösung kam eine Vorlage mit Rührer zum Einsatz. Als Vorratsgefäß für die Thiolacton-Lösung kam eine Vorlage mit Rührer zum Einsatz. Eine Standard-Rührwerksapparatur diente als Auffanggefäß für das Grignard-Additionsprodukt und dessen weitere Umsetzung mit CO₂. Zur Temperaturerfassung in der Apparatur wurde ein Pt100-Temperatursensor verwendet. Ein Stahlbehälter wurde als Vorratsgefäß für Lösungsmittel (Lösungsmittel ≡ LM) verwendet.

Die Edukt-Lösungen wurden mit geeigneten Pumpen durch eine Filtrationseinheit unter Kontrolle des Drucks und durch Rückschlagventile in die Mini/Mikroreaktoren gefördert. Die Thiolacton-Lösung wurde mit Hilfe eines Wärmeaustauschers temperiert.

Mit Dreiwegehähnen wurden zahlreiche Spülmöglichkeiten für Filter und Leitungen geschaffen, so daß bei Bedarf die Leitungen sowohl mit flüssigen als auch gasförmigen Medien gespült werden können. Beispielsweise ist auf diese Weise eine Spülung mit Inertgas oder mit Lösungsmittel, bzw. ein erforderlicher Druckausgleich möglich.

Der temperierbare Minireaktor bestand aus einer Misch- und Reaktionseinheit. Der Ausgang der Reaktionseinheit führte in die o. g. Rührwerksapparatur. Durch einen hinter dem Reaktor befindlichen Dreiwegehahn wurde die Möglichkeit einer Probenentnahme geschaffen.

Zur Steuerung und Aufzeichnung der Reaktionsparameter wurde eine geeignete Datenerfassungs- und Steuerungsanlage angeschlossen.

Die Durchführung des Verfahrens ist dem Fachmann mit einer entsprechend aufgebauten Apparatur, deren Einzelteile im Handel erhältlich sind, in der beschriebenen Weise möglich. Der Aufbau der Apparatur läßt sich dabei nach Bedarf, angepaßt an die jeweilig durchzuführende Reaktion, variieren.

Im folgenden werden zur Verdeutlichung der vorliegenden Erfindung Durchführungsbeispiele gebracht.

### Versuchsdurchführung:

### 1. Darstellung der Grignard- Verbindung.

Die 130 l Rührwerksapparatur wurde mehrfach evakuiert und mit Stickstoff belüftet. Es wurden 2.87 kg (116.9 mol) Magnesiumspäne (99%) vorgelegt und die Apparatur erneut mit Stickstoff inertisiert. Anschließend wurde mit 4.2 kg THF überschichtet, die Manteltemperatur auf 70°C eingestellt, und es wurden 4.0 kg einer Lösung von 7.5 kg (59.1 mol) 1,4-Dichlorbutan (>99%) in 38.8 kg THF zugegeben. Die Reaktion wurde durch Zugabe von ca. 50 ml 1,4-(Dichlormagnesium)-butan gestartet. Anschließend wurde die Manteltemperatur auf 60°C zurückgestellt. Nach Abreaktion der Startmenge wurde die restliche Lösung von 1,4-Dichlorbutan in THF innerhalb von 2 h 40 min so zugegeben, daß das Reaktionsgemisch stets unter Rückfluß siedete (nach 10 min Rührer angestellt). Es wurde weitere 90 min unter Rückfluß erhitzt (Manteltemperatur 80°C) und dann langsam auf 30°C abgekühlt. Dabei wurde die Lösung mit weiteren 55.9 kg THF verdünnt. Die Innentemperatur wurde dann konstant auf 28 bis 32°C gehalten. Man erhielt 110 kg Grignard-Lösung (c = 0.5 mol/l).

### 2. Darstellung der Thiolacton-Lösung.

In einer 200 l Vorlage mit Rührer wurden 11.2 kg (33.0 mol) Thiolacton (1) in 89.8 kg THF gelöst. Man erhielt 101 kg Lösung (c = 0.30 mol/l). Die Apparatur wurde unter Stickstoff gehalten.

### 3. Vorbereitungen und Durchführung der Reaktion im Statikmischer.

Alle Leitungen wurden zunächst mit wasserfreiem THF gespült und getrocknet und mit Stickstoff inertisiert. Nach Vorlegen von 65 THF wurde die Rührwerksapparatur evakuiert und mit Kohlendioxid belüftet. Die 800 l RWA, die Mischeinheit und der Wärmetauscher für die Thiolacton-Vorkühlstrecke wurden mit Sole gekühlt (ca. -14 bis -12°C).

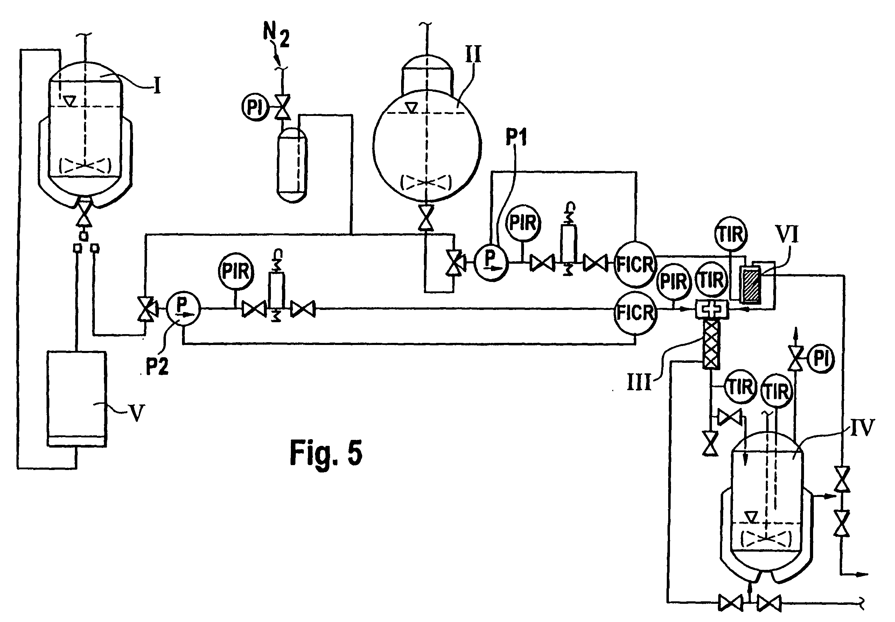

Ein Fließschema der verwendeten Anlage ist in Fig. 5 dargestellt.

Wie beschrieben, ist die Anlage aus folgenden Komponenten Aufgebaut:
- Rührwerksapparatur mit Rührer, 130 l, Email, mit Temperierung
- Rührwerksapparatur mit Rührer, 800 l, Email, mit Temperierung
- Glasvorlage mit Rührer, 200 l
- Seitz-Einschicht-Druckfilter, Edelstahl, 120 l, Ø = 65 cm
- 2 Zahnradpumpen, Edelstahl, Gather Industries
- Pall-Kerzenfilter, Edelstahlgewebe, 50 µm, 2.5 l Volumen
- Edelstahlfass, druckbeständig, 100 l
- Wärmeaustauscher aus 6 mm Stahlrohr, Wendel 3m Länge
- 6 mm und 100 mm Stahlrohre
- Swadgelok-Verbinder, -Hähne und -Schläuche

Für die Meß- und Regelungstechnik wurden neben den Standard-Sensoren der 800 l Rührwerksapparatur folgende Sensoren eingesetzt:
Für
- T-Messung hinter der gesamten Mischeinheit
- T-Messung der Sole nach der Mischeinheit und vor dem Vorkühler für Thiolacton
wurden
=> Pt100-Sensoren, 6 mm Stahlgehäuse, Fa. Cowie AG (Schweiz)
=> Pt100-Sensoren, 6 mm Teflongehäuse, Fa. Cowie AG (Schweiz)
eingesetzt
und für die Druckmessungen
- 2x P-Messung hinter der Zahnradpumpe vor dem Filter
- P-Messung vor dem Mischer in der Grignard-Leitung
wurden
=> Druckmeßumformer P41, [Stahlmembran, M20 x 1.5, 0-10 bar Absolutdruck, Fa. PMH (Prozeß- und Maschinen-Automation GmbH (Philips, D)]
eingesetzt.

Stoffströme wurden gemessen:
- 2x Coriolis Massedurchflußmesser
   => Massflo® Typ Mass 3000/2100 Ex (Meßumformer und -aufnehmer), Fa. Danfoss Antriebs- und Regeltechnik GmbH

Der Grignard-Strom wurde auf 30 kg/h eingestellt (3 kg wurden ohne Umsetzung mit Thiolacton in die 800 l RWA gepumpt) und der Thiolacton-Strom mit Hilfe eines Faktors angepasst, (Dichteunterschied). Diese Betriebseinstellung (30 kg/h) wurde bis zum vollständigen Verbrauch der Grignard-Lösung beibehalten. Der CO₂-Strom mußte auf ca. 25 l/h eingestellt werden.

Nach dem Verbauch von
99.6 kg (≡ 32.5 mol Thiolacton) Thiolacton-Lösung und ca. 110 kg Grignard-Lösung wurde die Kühlung abgestellt, und es wurde anschließend noch weitere 30 min CO₂ durch die Apparatur geleitet. Die entstandene Suspension wurde über Nacht gerührt.

Unter heftigem Rühren wurde auf ca. 42 °C erwärmt und dabei auf 400 mbar evakuiert. Es wurde THF soweit abdestilliert, bis der Rückstand sehr zähflüssig wurde. Es wurden 30 l Xylol zugegeben und weiter THF entfernt. Nach der Zugabe von weiteren 30 l Xylol wurden langsam unter Rühren 66 kg 30%ige H₂SO₄ zugegeben, wobei sich das Gemisch bis auf T = 67 °C erwärmte. Es wurde weitere 120 min bei 62 - 70 °C Innentemperatur mit maximaler Rührerdrehzahl nachgerührt und anschließend die Manteltemperatur auf 50 °C zurückgestellt. Nach Erreichen der gewünschten Temperatur wurde der Rührer abgestellt. Die wäßrige Phase trennte man bei ca. 65°C ab, und die organische Phase wurde auf 30°C abgekühlt. Durch Zugabe von ca. 45 l 1 N NaOH wurde unter heftigem Rühren der pH-Wert auf pH = 9.37 eingestellt. Der Rührer wurde abgestellt, und die Phasen wurden nach 1 h getrennt (Xylol 1). Die wäßrige Phase wurde in die Apparatur zurückgegeben, und es wurden 60 l Xylol zugeben. Der pH-Wert wurde mit ca. 4 l 25%iger Salzsäure auf pH = 6.81 eingestellt, die Phasen wurden getrennt (ca. 30 min), und man erhielt das Produkt in Xylol (Xylol 2).

Teilmengen der beiden Xylol-Phasen wurden azeotrop im Vakuum vom Lösungsmittel und Wasser befreit. Aus den erhaltenen Massen wurden die Gesamtausbeuten bestimmt.

| | |
|---|---|
| Xylol 1 | 2.07 kg (Nebenprodukte) |
| Xyloi 2 | 12.14 kg (Hauptprodukt, 88.2% Ausbeute) |

Während der Durchführung dieses Versuchs betrug die Temperatur am Ausgang des Mischers ca. 2h 31 - 33°C und den Rest der Zeit ca. 28-29 °C.

Es konnte ein Druckanstieg von ca. 1 bar im gesamten System beobachtet werden. Die Temperatur der Grignard-Lösung wurde auf 30°C eingestellt. Das Regelverhalten der Grignard-Pumpe stieg um ca. 10 Einheiten an. Der maximale Druck im System betrug ca. 4.5 bar am Ende des Versuchs.

Die Analyse der Nebenprodukte ergab, daß es sich um ein korrespondierendes Diolefin, die Butyliden-Verbindung und das Reduktionsprodukt der gewünschten Verbindung (3) sowie um Ureidthiol handelt.

In gleicher Weise läßt sich 4-[4-(trans-4-Pentylcyclohexyl)-1-cyclohexen-1-yl]-1-trifluormethoxybenzol (CCP-5-OCF3-enyl) (5) unter Einsatz von n-Butyllithium herstellen. Als Edukte wurden 4-Brom-trifluormethoxybenzol (3) und 4-(4-Pentylcyclohexyl)-cyclohexan-4-on (4) eingesetzt. Nach der nachgeschalteten Hydrolyse wird das gewünschte Produkt (5) erhalten.

Weiterhin läßt sich Cyclohexyl-cyclohexan-3-on mit 3,4,5-Trifluor-1-brommagnesiumbenzol bei Temperaturen zwischen 0 bis 60 °C umsetzen. Auch hier kann die Verweilzeit von mehreren Stunden auf weniger als 10 s verkürzt werden. Im Gegensatz zu der oben beschriebenen Reaktion lassen sich die Ausbeute und damit die Produktqualität durch Druck- und Temperaturerhöhung optimieren, wobei jedoch zu beachten ist, daß die Grignard-Verbindung oberhalb von 100 °C zur Zersetzung in einer stark exothermen Reaktion neigt und der Siedepunkt von THF bei 65 °C liegt. Durch die inhärente Sicherheit der Mikromischer kann hier jedoch trotzdem bei höheren Temperaturen gearbeitet werden, so daß durch die vorliegende Erfindung eine sonst nicht mögliche Reaktionsführung ermöglicht wird.

Darstellungen:
**Fig.1**
   Mini-Statikmischer wie bereits in DE 19746583 A1 beschrieben
**Fig. 2**
   T-Stück-Mischer 1
   Vereinfachter Mischer, in dem die Edukte (1) und (2) in einem temperierten T-Stück (III) mit Hilfe von dünnen Leitungen mit einem Innendurchmesser von 2 mm, die von einem Kühlmantel (4) umgeben sind, zusammengeführt werden. Der Leitungsinnnendurchmesser beträgt innerhalb des Kühlmantels 2 mm und außerhalb 4 mm.
**Fig. 3**
   T-Stück-Mischer 2
   Statischer Mischer wie in Fig. 2 mit einem Innendurchmesser der Leitungen im T-Stück (III) von 4 mm, worin jedoch nach dem Zusammenführen der Edukte (1) und (2) das Reaktionsgemisch durch einen Statikmischer mit zopfförmigen Leitungen (5) mit einem Innendurchmesser von ca. 4mm geführt wird.
**Fig. 4**
   Grundlegender Aufbau einer Anlage:
   Komponenten der Anlage sind:
   Vorratstanks bzw. Rührbehälter (I) und (II) enthaltend Edukte 1 und 2 Reaktor (III)
   Vorratstank bzw. Rührbehälter (IV) für das Reaktionsgemisch
   Pumpen P1 und P2
**Fig. 5**
   Aufbau der für die Versuche verwendeten Anlage:
   Rührwerksapparatur mit Rührer, Email, mit Temperierung (I)
   Rührwerksapparatur mit Rührer, Email, mit Temperierung (IV)
   Glasvorlage mit Rührer, (II)
   Seitz-Einschicht-Druckfilter, Edelstahl, (V)
   2 Zahnradpumpen, (P1, P2)
   Wärmeaustauscher (VI)
   Pall-Kerzenfilter; Edelstahlfass, druckbeständig; 6 mm und 100 mm Stahlrohre; Swadgelok-Verbinder, -Hähne und -Schläuche.

## Patentansprüche

1. Verfahren zur Durchführung von Reaktionen von Carbonylverbindungen mit metallorganischen Reagenzien, **dadurch gekennzeichnet, dass** die Lösungen
a) eines metallorganischen Reagenzes ausgewählt aus der Gruppe der Grignardreagenzien und lithiumorganischen Verbindungen, gelöst in einem aprotischen nukleophilen Lösungmittel und
b) einer Carbonylverbindung aus der Gruppe der aliphatischen, cycloaliphatischen und heterocyclischen Ketone, gelöst in einem aprotischen nukleophilen Lösungmittel
bei einer konstanten Temperatur zwischen -40 bis +120 °C, in einer Verweilzeit von τ < 10 s
unter Kontrolle des Drucks,
gegebenenfalls nachdem die Eduktlösungen aus a) und b) mit Pumpen durch eine Filtrationseinheit geführt worden sind,
in einem kontinuierlich betriebenen Mini/Mikroreaktor intensiv vermischt werden, wobei es sich bei dem Mini/Mikroreaktor um einen temperierten, plattenförmigen Mikromischer handelt, worin die Eduktlösungen in einem 180°-T-Stück oder in einem Winkel von 30 bis 150 ° zusammengeführt und intensiv in, gegebenenfalls zopfförmigen, dünnen Leitungen vermischt werden
und gegebenenfalls das resultierende Vorprodukt durch eine nachgeschaltete, sehr dünne, temperierte Verweilstrecke geführt wird.

2. Verfahren gemäß Anspruch 1, **dadurch gekennzeichnet, dass** ein metallorganisches Reagenz aus der Gruppe
1,4-Dichlor-magnesiumbutan, 3,4,5.Trifluor-1-brommagnesiumbenzol und n-Butyllithium und eine
Carbonylverbindung ausgewählt aus der Gruppe 4-(4-Pentylcyclohexyl)-cyclohexan-4-on (4) und (+)-cis-1,3-Dibenzyl-hexahydro-1H-thieno[3,4-d]imidazol-2,4-dion
umgesetzt werden, wobei ein Lösungsmittel ausgewählt aus der Gruppe Tetrahydrofuran, Diethylether, Dioxan und Dibutylether verwendet wird.

3. Verfahren gemäß Anspruch 1 und/oder 2, **dadurch gekennzeichnet, dass** das aus dem Mini/Mikromischer bzw. der Verweilstrecke austretende Reaktionsgemisch in einer nachgeschalteten Reaktion umgesetzt wird und das erhaltene Produktgemisch aufgearbeitet wird.

4. Verfahren gemäß der Ansprüche 1 bis 3, **dadurch gekennzeichnet, dass** die Lösungen a) und b) in den Reaktor (III) geführt werden, nachdem sie Vortemperierstrecken durchlaufen haben.

5. Verfahren gemäß Anspruch 3, **dadurch gekennzeichnet, dass** das Reaktionsgemisch durch hintereinandergeschaltete Reaktoren geführt wird, worin nachgeschaltete Reaktionen durchgeführt werden.

6. Verfahren gemäß der Ansprüche 1 bis 3 **dadurch gekennzeichnet, dass** das aus dem Mini/Mikromischer austretende Reaktionsgemisch mit einer nachgeschalteten Pumpe in einen Vorratstank bzw. Rührreaktor oder Mini/Mikroreaktor gefördert wird.

7. Verfahren gemäß der Ansprüche 1 bis 6, **dadurch gekennzeichnet, dass** als nachgeschaltete Reaktion eine Umsetzung mit Kohlendioxid oder eine Hydrolyse erfolgt.

## Claims

1. Process for carrying out reactions of carbonyl compounds with organometallic reagents, **characterised in that** the solutions
a) of an organometallic reagent selected from the group of the Grignard reagents and organolithium compounds, dissolved in an aprotic nucleophilic solvent and
b) a carbonyl compound from the group of the aliphatic, cycloaliphatic and heterocyclic ketones, dissolved in an aprotic nucleophilic solvent
are mixed intensively in a continuously operated mini/microreactor at a constant temperature between -40 and +120°C, in a residence time of τ < 10 s
with control of the pressure,
optionally after the starting-material solutions from a) and b) have been fed through a filtration unit by means of pumps,
where the mini/microreactor is a temperature-controlled, plate-shaped micromixer in which the starting-material solutions are combined in a 180° T-piece or at an angle of 30 to 150° and mixed intensively in optionally plait-shaped, thin lines
and the resultant precursor is optionally fed through a downstream, very thin, temperature-controlled residence zone.

2. Process according to Claim 1, **characterised in that** an organometallic reagent from the group
1,4-dichlorobutylmagnesium, 3,4,5-trifluoro-1-bromophenylmagnesium and n-butyllithium and a
carbonyl compound selected from the group 4-(4-pentylcyclohexyl)cyclohexan-4-one (4) and (+)-cis-1,3-dibenzylhexahydro-1H-thieno[3,4-d]-imidazole-2,4-dione
are reacted, where a solvent selected from the group tetrahydrofuran, diethyl ether, dioxane and dibutyl ether is used.

3. Process according to Claim 1 and/or 2, **characterised in that** the reaction mixture leaving the mini/micromixer or the residence zone is reacted in a downstream reaction, and the resultant product mixture is worked up.

4. Process according to Claims 1 to 3, **characterised in that** solutions a) and b) are fed into the reactor (III) after they have passed through preliminary temperature-control zones.

5. Process according to Claim 3, **characterised in that** the reaction mixture is fed through reactors connected in series, in which downstream reactions are carried out.

6. Process according to Claims 1 to 3, **characterised in that** the reaction mixture leaving the mini/micromixer is conveyed into a storage tank or stirred-tank reactor or mini/microreactor by means of a downstream pump.

7. Process according to Claims 1 to 6, **characterised in that** a reaction with carbon dioxide or a hydrolysis is carried out as downstream reaction.

## Revendications

1. Procédé pour mettre en oeuvre des réactions de composés carbonyle avec des réactifs organométalliques, **caractérisé en ce que** les solutions
a) d'un réactif organométallique choisi parmi le groupe des réactifs de Grignard et des composés d'organolithium, dissout dans un solvant aprotique nucléophile et
b) d'un composé carbonyle pris parmi le groupe des cétones aliphatiques, cycloaliphatiques et hétérocycliques, dissout dans un solvant aprotique nucléophile
sont mélangées de façon intense dans un mini/microréacteur actionné en continu
à une température constante entre -40 et +120°C, selon un temps de résidence de τ < 10 s
avec contrôle de la pression,
en option après que les solutions de matériau de départ issues de a) et de b) ont été alimentées au travers d'une unité de filtration au moyen de pompes,
où le mini/microréacteur est un micromélangeur en forme de plaque contrôlé en température dans lequel les solutions de matériau de départ sont combinées dans une pièce en T à 180° ou selon un angle de 30 à 150° et sont mélangées de façon intense dans en option des lignes minces en forme de tresse
et le précurseur résultant est en option alimenté au travers d'une zone de résidence aval très mince contrôlée en température.

2. Procédé selon la revendication 1, **caractérisé en ce qu'**un réactif organométallique pris parmi le groupe
1,4-dichlorobutylmagnésium, 3,4,5-trifluoro-1-bromophénylmagnésium et n-butyllithium et un
composé de carbonyle choisi parmi le groupe 4-(4-pentylcyclohexyl)-cyclohexane-4-one (4) et (+)-cis-1,3-dibenzylhexahydro-1H-thiéno[3,4-d]-imidazole-2,4-dione
sont amenés à réagir, où un solvant choisi parmi le groupe tétrahydrofurane, éther diéthylique, dioxane et éther dibutylique est utilisé.

3. Procédé selon la revendication 1 et/ou 2, **caractérisé en ce que** le mélange de réaction qui quitte le mini/micromélangeur ou la zone de résidence est amené à réagir au niveau d'une réaction aval, et le mélange de produit résultant est élaboré.

4. Procédé selon les revendications 1 à 3, **caractérisé en ce que** des solutions a) et b) sont alimentées dans le réacteur (III) après qu'elles sont passées au travers de zones de contrôle de température préliminaires.

5. Procédé selon la revendication 3, **caractérisé en ce que** le mélange de réaction est alimenté au travers de réacteurs connectés en série, dans lesquels des réactions aval sont mises en oeuvre.

6. Procédé selon les revendications 1 à 3, **caractérisé en ce que** le mélange de réaction qui quitte le mini/micromélangeur est convoyé dans un réservoir de stockage ou un réacteur à réservoir agité ou un mini/microréacteur au moyen d'une pompe aval.

7. Procédé selon les revendications 1 à 6, **caractérisé en ce qu'**une réaction avec du dioxyde de carbone ou une hydrolyse est mise en oeuvre en tant que réaction aval.
